# EUROPEAN PATENT APPLICATION

(11) **EP 4 356 896 A1**
(43) Date of publication of application: **24.04.2024**
(21) Application number: 23203762.2
(22) Date of filing: 16.10.2023
(51) Int. Cl.: A61K 8/02, A61K 8/34, A61K 8/365, A61K 8/44, A61K 8/46, A61Q 5/02, A61Q 5/12, A61Q 19/10, C11D 1/94, C11D 3/00

(54) **COMPOSITION COMPRISING SURFACTANT PREPARED WITH CARBON FROM CARBON CAPTURE**

(30) Priority: 18.10.2022 US 202263417087 P; 16.12.2022 EP 22214231
(71) Applicant: Unilever IP Holdings B.V., 3013 AL Rotterdam (NL); Unilever Global IP Limited, Wirral, Merseyside CH62 4ZD (GB)
(72) Inventor: GHATLIA, Naresh, Dhirajlal, 6708 WH Wageningen (NL); VASUDEVAN, Tirucherai, Varahan, 6708 WH Wageningen (NL)
(74) Representative: Fijnvandraat, Arnoldus

(57) **Abstract**

The invention is directed to a wash composition suitable for topical application that comprises a surfactant having carbon obtained from carbon capture. The composition can have a pH that is from the natural pH of skin to as high as 7.25. The composition is also substantially free of parabens and can be formulated with less than 25 ppm heterocyclic impurities.

## Description

### Field of the invention

The present invention is directed to a wash composition comprising a surfactant that includes carbon obtained from carbon capture. More particularly, the composition comprises anionic surfactant, nonionic surfactant or both that includes carbon obtained from carbon capture. The anionic surfactant is from 10 to 100% by weight alkyl ether sulfate, an acyl isethionate or a mixture thereof based on total weight of anionic surfactant. The nonionic surfactant is from 10 to 100% alcohol ethoxylate based on total weight of nonionic surfactant. At least 10% by weight of the anionic surfactant and/or the nonionic surfactant used comprises carbon obtained from carbon capture, yielding a surprisingly stable wash composition having an improved environmental footprint.

### Background of the invention

Personal cleansing processes characteristically involve the use of surfactant comprising compositions (i.e., wash compositions) and water to topically generate consumer desired lather and to rinse off the compositions typically applied to the face, hands, hair and/or body. Such activities are performed daily, often more than once a day, and especially, as it relates to the hands and face of a consumer. In the current post pandemic environment, personal cleansing activities are highly urged to prevent the spread of germs and to reduce the chance of getting common ailments like a cold and influenza. Ideally, washing can be optimized when using stable products that are mild, have excellent lather characteristics and that rinse off with relative ease. Furthermore, many consumers desire products that not only perform well but that are also better for the environment, including products using recycled plastics and/or ingredients recovered from the atmosphere, especially greenhouse gases.

The present invention, therefore, is directed to a stable wash composition that uses ingredients that benefit the environment. The wash composition is suitable to have a pH at the natural pH of skin yet can be stabilized with a preservative that includes phenoxyethanol (with or without capryloyl glycine and/or undecylenoyl glycine), iodopropynyl butylcarbamate, benzoic acid (and/or a derivative of benzoic acid) as well as mixtures thereof. Such a wash composition can be formulated substantially free of isothiazolinones, hydantoins and parabens, can be formulated to comprise less than 25 ppm heterocyclic impurities, especially those classified as ethers, like 1,4-dioxane and can be formulated to comprise reduced amounts of surfactant made with palm kernel oil to aid in preserving the planet's ecosystem. The composition can be mild, lather well, be surprisingly stable (e.g., free of syneresis and discoloration), easily rinsed off and odor free even after being stored for 2 months at 45°C.

### Additional Information

Efforts have been disclosed for making wash compositions. In U.S. Patent No. 11,147,757, a composition comprising a surfactant system consisting essentially of an anionic surfactant and a zwitterionic surfactant; a preservative; a pH modifier; and a rheology modifier is described.

Other efforts have been disclosed for making wash compositions. In U.S. Patent No. 5,683,683, body wash compositions with anionic cleansing surfactant, polymeric cationic conditioning compound and a quaternized phosphate ester in an aqueous carrier are described.

Even other efforts have been disclosed for making wash compositions. In U.S. Patent No. 7,375,063, compositions with an acrylate copolymer emulsion, anionic surfactant and amphoteric surfactant are described.

None of the additional information above describes a wash composition suitable to have anionic surfactant, nonionic surfactant or both that include(s) carbon obtained from carbon capture.

### Summary of the invention

In a first aspect, the present invention is directed to a wash composition comprising:
(a) anionic surfactant, nonionic surfactant or both;
(b) amphoteric surfactant, zwitterionic surfactant or both
(c) 0 to 55% by weight polyol; and
(d) water
wherein the anionic surfactant is from 10 to 100% by weight alkyl ether sulfate, acyl isethionate or a mixture thereof based on total weight of anionic surfactant, and the nonionic surfactant is from 10 to 100% alcohol ethoxylate based on total weight of nonionic surfactant, and further wherein at least 10% by weight of the total weight of anionic and/or nonionic surfactant used comprises carbon derived from carbon capture, the wash composition having a water activity under 1, a pH from 4 to 7.25, and preferably 4.2 to 7.25, and most preferably, from the pH of skin to 7.25 (or from 4.5 to 6.85 or from 5.0 to 6.75 or from 5.5 to 6.5).

In a second aspect, the present invention is directed to a wash composition comprising:
(a) anionic surfactant, nonionic surfactant or both;
(b) amphoteric surfactant, zwitterionic surfactant or both
(c) 0 to 55% by weight polyol;
(d) 0.01 to 15% by weight of a polyether; and
(e) water
wherein:
i) the anionic surfactant is from 10 to 100% by weight alkyl ether sulfate, acyl isethionate or a mixture thereof based on total weight of anionic surfactant, and the nonionic surfactant is from 10 to 100% alcohol ethoxylate based on total weight of nonionic surfactant;
ii) at least 10% by weight of the total weight of anionic and/or nonionic surfactant used comprises carbon derived from carbon capture;
iii) at least 10% by weight of the total weight of polyether in the wash composition has carbon derived from carbon capture; and
iv) the wash composition has a water activity under 1, a pH from 4 to 7.25, and preferably 4.2 to 7.25, and most preferably, from the pH of skin to 7.25 (or from 4.5 to 6.85 or from 5.0 to 6.75 or from 5.5 to 6.5).

In a third aspect, the present invention is directed to the use of anionic surfactant, nonionic surfactant or both to make a shampoo, shampoo and conditioner, hand wash, face wash or body wash, wherein at least one of the anionic surfactant and nonionic surfactant comprises carbon derived from carbon capture.

In a fourth aspect, the present invention is directed to the use of a polyether, and anionic surfactant, nonionic surfactant or a mixture thereof to make a shampoo, shampoo and conditioner, hand wash, face wash or body wash, wherein at least one of the anionic surfactant and nonionic surfactant comprise carbon derived from carbon capture and the polyether comprises carbon derived from carbon capture.

In a fifth aspect, the present invention is directed to a method for making a wash composition comprising, in no particular order, the steps of combining to produce a mixture having:
a) at least one anionic surfactant and/or nonionic surfactant comprising carbon derived from carbon capture, the anionic surfactant comprises alkyl ether sulfate, an acyl isethionate or a mixture thereof and the nonionic surfactant comprises alcohol ethoxylate;
b) amphoteric or zwitterionic surfactant or both;
c) 0 to 55% by weight polyol;
d) 0 to 15% by weight of a polyether; and
e) water
wherein the resulting mixture is mixed or sheared to produce a homogeneous emulsion and further wherein at least one of the anionic surfactant and/or the nonionic surfactant comprises carbon derived from carbon capture, preferably both, and the polyether comprises carbon derived from carbon capture.

In a sixth aspect, the present invention is directed to the use of (or method of washing or treating with) a shampoo, shampoo and conditioner, hand wash, face wash or body wash comprising an anionic surfactant and/or nonionic surfactant comprising carbon derived from carbon capture, and optionally, polyether comprising carbon derived from carbon capture to wash hair or skin or both on the body.

In a seventh aspect, the present invention is directed to the use of a surfactant and/or other ingredient comprising carbon derived from carbon capture to make a fast-moving cosmetic consumer product.

In an eighth aspect, the present invention is directed to a wash composition that is made by a process that uses as an ingredient a surfactant or polyether or both that includes carbon derived from carbon capture.

All other aspects of the present invention will more readily become apparent from the description and examples which follow.

Mild on skin and hair means the skin and hair do not feel dry after multiple uses as determined by trained panelists. Easily rinsed off means the wash composition does not leave a residue feeling, sticky or draggy feeling after rinsing with water. Stable, as used herein means the wash composition is free of discoloration (i.e., browning or yellowing), odor and syneresis after visual inspection and after being stored for 2 months at 45°C. Stable also includes having antimicrobial stability, and therefore, at least a 3 log bacteria kill at 7 days and at least a 2 log yeast and mold kill at 7 days, all as described in General Chapter 51 of the United States Pharmacopeia (USP) and maintaining consumer desired fragrance characteristics. Skin, as used herein, is meant to include skin on the arms (including underarms), face, feet, neck, chest, hands, legs, buttocks, back and scalp. Naturally derived preservative, as used herein, means a preservative that, for example, can be isolated from a plant like the monoterpenoid phenol, thymol, isolated from the oil of thyme extracted from *Thymus vulgaris.* Other natural preservatives suitable for use comprise benzoic acid and/or a derivative thereof isolated from fruits like apples and strawberries. Wash composition, as used herein, is meant be an end use composition ready to use by a consumer including a composition ready for topical application like a shampoo, shampoo and conditioner, face wash (including make-up remover) or body wash, including bars, liquids, compositions impregnated on a substrate like a nonwoven substrate, concentrates for dilution and compositions that are anhydrous and hydratable. In one embodiment, the composition is a liquid personal wash composition, and most preferably, a liquid body wash. In another embodiment, the composition is a shampoo composition or shampoo and conditioner. In even another embodiment the composition is a face or hand wash composition with or without antibacterial benefit agents where the face wash includes anti-acne washes. Still further, the wash composition can be a 3-in-1 composition to wash and condition hair and be used as a body wash. As hereinafter described, the wash composition of the present invention may optionally comprise skin benefit ingredients added thereto such as emollients, vitamins and/or derivatives thereof, resorcinols, retinoic acid precursors, colorants, moisturizers, sunscreens, mixtures thereof or the like. The skin benefit ingredients may be water or oil soluble. Sulfate-based means having an SO₄ group such as the S containing group or moiety in sodium lauryl ether sulfate. Mild surfactant system means less than 11.0% by weight, and preferably, less than 9%, and most preferably, less than 8%, (or less than 6% or 5% or from 0.75 to 4.5% by weight) sulfate-based surfactant. Substantially free of means less than 0.5% by weight, and preferably, less than 0.3% by weight, and most preferably, less than 0.15% by weight (or less than 0.1% or 0.05% or 0.04 to 0.01 % or 0.0% (none) by weight) based on total weight of the wash composition. Lathers well is based on panellist perception and observation whereby the composition of the present invention can foam in a manner that is consistent with wash compositions having surfactants and other ingredients not prepared with carbon derived from carbon capture. Viscosity, as used herein, means taken with a Brookfield RV5 suitable for wash compositions, at 20 rpm for 30 seconds and at 25°C. In still another embodiment, the wash composition of this invention is a non-therapeutic and cosmetic composition which is a liquid personal wash, bar composition, shampoo or shampoo and conditioner to cosmetically treat and/or clean hair and/or skin with use. In the absence of explicitly stating otherwise, all ranges described herein are meant to include all ranges subsumed therein. The term comprising is meant to encompass the terms consisting essentially of and consisting of. For the avoidance of doubt, and for illustration, a composition of this invention comprising an anionic and zwitterionic surfactant, water, sodium benzoate and polyol is meant to include a composition consisting essentially of the same and a composition consisting of the same. Except in the operating comparative examples, or where otherwise explicitly indicated, all numbers in this description indicating amounts or ratios of materials or conditions and/or physical properties of materials and/or use are to be understood as modified by the word "about" and "including all ranges subsumed therein". In another embodiment of the invention, a liquid wash composition consistent with this invention is one having a viscosity from 750 to 22,000 cps, and preferably, from 1,000 to 17,000 cps, and most preferably, from 2,000 to 16,000 cps or from 2,000 to 11,000 cps. In an embodiment of the invention, the viscosity is from 1,000 to 5,000 or from 1,900 to 4,250 cps when the wash composition is a hand wash composition. Comprises carbon obtained from carbon capture means there is carbon in the surfactant or ingredient (in the invention in the hydrocarbon chain and/or ethoxylate portion of anionic surfactant, nonionic surfactant and/or polyether as herein defined) not directly from fossil fuel or derived from vegetable sources, but recovered and reused, where the same further means any amount of carbon from carbon capture and therefore more than 0.0% of the carbon in the surfactant and polyether.

### Detailed Description of the Preferred Embodiments

In one embodiment, the personal wash composition of the present invention can include anionic surfactants comprising alkyl ether sulfates and/or nonionic surfactants classified as alcohol ethoxylates that are surfactants with 1 to 7 ethoxylate groups, EO groups, preferably 1 to 6, and most preferably, 1 to 5 or 1 to 4 or 1 to 3, and even more preferably, 1 or 2 ethoxylate groups. Optional ingredients like polyethers, including polyethylene glycol, may also be used in the compositions in order to impact cleaning performances and/or sensory benefits desired by consumers. The anionic and nonionic surfactants as well as such optional ingredients (i.e., ethoxylated ingredients like polyethers, preferably weight average molecular weight under 20,000) are suitable to be prepared with recycled carbon to yield wash compositions that surprisingly are simultaneously desired by consumers and that can provide a positive impact on the environment, including the earth's ecological footprint.

Carbon Capture is highly appealing to reduce carbon emissions and to minimize global warming. The reduction of carbon emissions is especially appealing to improve the overall quality of life for mankind, as well as plants, trees, and wildlife. Minimizing and even avoiding the reliance of non-renewable virgin fossil fuels, known as "black" carbon, has become a goal for many environmentally conscious countries and companies. Use of renewable and recycled carbon sources (i.e., carbon emissions) that include carbon captured from the air or from industrial emissions, like those originating from agricultural and waste management processes as well as power generation, mining, steel, and cement generation processes (or the like) to create new ingredients for products is of interest to many.

In general, and within carbon emissions, carbon dioxide gas, for example, can be recovered and combined with various bacterial cultures (and/or some yeasts) to convert the gases into ethanol. What has been publicized, are methods that capture air (e.g., via electrolysis) and other industrial and waste gases where the collected gases are compressed, cleaned, subject to fermentation and finally separated to produce ingredients or materials including ethanol. Entities such as LanzaTech or Lanza Tech with partners like Twelve are examples of entities reducing the reliance on fossil fuels, producing ethanol by converting CO₂ to CO where the resulting CO can subsequently be subjected to a small continuous stirred tank reactor (CSTR), like those from Lanza Tech, to convert CO into ethanol. An illustrative process for making hydrocarbons from industrial gases comprising CO₂ is described in world patent application WO2012/058508.

In particular, carbon capture refers to the capture or sequestration of single carbon (C₁) molecules (e.g., carbon monoxide, carbon dioxide, methane or methanol). By capturing such carbon molecules, they are removed from or prevented from entering the environment. Carbon sourced from carbon capture contrasts with carbon from virgin fossil fuels (crude oil, natural gas, etc.) in that captured carbon has been used at least once, and for example, captured carbon may have been burned to produce energy and is captured to enable a second use of such carbon. Carbon (i.e., captured) is recycled. In contrast, carbon from virgin fossil fuels has been extracted for a first and single purpose. Captured utilized carbon is suitable for use again, leading to less carbon in the atmosphere and reduced use of virgin fossil fuels. The carbon captured may be in any physical state, but preferably is a gas.

Carbon capture may be point source carbon capture (stationary or mobile) or direct carbon capture. Direct carbon capture refers to capturing carbon from the air, where it is significantly diluted with other atmospheric gases. Point source carbon capture refers to the capture of carbon at the point of release into the atmosphere. Point source carbon capture may be implemented for example at steel work facilities, fossil fuel or biomass energy plants, ammonia manufacturing facilities, cement factories or the like. These are examples of stationary point source carbon capture origins. Alternatively, the point source carbon capture may be mobile, and for example, attached to a vehicle to capture carbon in exhaust gases. Point source carbon capture tends to be preferable due to the efficiency of capturing the carbon in a high concentration. In an embodiment of the invention, therefore, the carbon used is preferably captured from a point source origin.

Methods are known for capturing carbon from industrial processes, examples include:
- Capturing carbon from flue gasses following combustion, often an option at fossil fuel power plants.
- Capturing carbon pre-combustion. In such processes, fossil fuels are typically partially oxidized and syngas comprising carbon monoxide, hydrogen and carbon dioxide is recovered. The carbon monoxide recovered is reacted with water (steam) to produce carbon dioxide and hydrogen. The carbon dioxide can be separated, and the hydrogen used as fuel.
- Oxy-fuel combustion, in which fuel is burned in oxygen rather than air. In such a process, the resulting flue gas consists primarily of carbon dioxide and water vapor. The water is separated out and the carbon dioxide collected.

After capturing a source of carbon, the carbon molecules are isolated from other chemicals with which they may be mixed in with, including for example, oxygen, water vapor, nitrogen, argon, mixtures thereof or the like. In some point source processes, this capturing step may not be required since a pure source of carbon may be captured. Separation may involve biological separation (as noted), chemical separation, absorption, adsorption, gas separation membranes, diffusion, rectification or condensation or any combination thereof.

A common method of separation is absorption or carbon scrubbing with amines. Carbon dioxide is absorbed onto a metal-organic framework or through liquid amines, leaving a low carbon gas which can be released into the atmosphere. The carbon dioxide can be removed from the metal-organic framework or liquid amines, for example, by using heat, pressure, or both.

Carbon (C₁, single carbon) molecules sourced from carbon capture and separated from other gases are available commercially from suppliers, including Ineos and Climeworks. Capturing carbon directly from the air may for example involve passing air over a solvent which physically or chemically binds to the single carbon molecules. Solvents include strongly alkaline hydroxides such as potassium or sodium hydroxide whereby air may be passed over a solution of potassium hydroxide to form a solution of potassium carbonate. The resulting carbonate solution is purified and separated to provide pure CO₂ gas. This method may also be employed in point source capture.

Subsequent to recovering the carbon molecules, such molecules can then be transformed into useful ingredients and surprisingly suitable for use to make stable fast-moving cosmetic consumer goods, like a shampoo, shampoo and conditioner, hand wash, face wash (including make-up remover) or body wash as described herein.

Various methods may be used to convert the captured single carbon molecules to useful ingredients. The methods may involve chemical process or biological processes or both, including, as noted, microbial fermentation.

Preferably the singe carbon molecules are transformed into:
i. short chain intermediates like methanol, ethanol, ethylene and/or ethylene oxide; or
ii. hydrocarbon intermediates like saturated and unsaturated hydrocarbon chains (e.g., alkanes, alkenes, etc.).

These resulting materials are suitable to be converted further to make components of surfactants, via well-known chemical reactions, and for example, via chain growth reaction methods to increase carbon length (i.e., molecular weight) to yield desired hydrocarbons. Preferably the single carbon molecules are transformed into short chain intermediates, and more preferably, ethanol, ethylene and/or ethylene oxide.

As to the short chain intermediates, a suitable example of transformation utilizes a process in which a reactor converts carbon dioxide and water with electricity to methanol or ethanol and oxygen. A known example of this process is provided by Opus 12.

An alternate means for such transformation is the conversion of carbon dioxide to ethanol using a catalyst of copper nanoparticles embedded in carbon spikes, as described by Oak Ridge National Laboratory in Oak Ridge, Tennessee.

Even another alternative for such transformation is biological transformation whereby fermentation of the single carbon molecule by micro-organisms (such as C₁-fixing bacteria) yields useful chemicals/compounds. The ability of microorganisms to grow on CO as a sole carbon source was first discovered in 1903. This was later determined to be a property of organisms that use the acetyl coenzyme A (acetyl CoA) biochemical pathway of autotrophic growth (also known as the Woods-Ljungdahl pathway and the carbon monoxide dehydrogenase / acetyl CoA synthase (CODH/ACS) pathway). Many anaerobic organisms including carboxydotrophic, photosynthetic, methanogenic and acetogenic organisms have been shown to metabolize CO to various end products, namely CO₂, H₂, methane, n-butanol, acetate and ethanol. In an embodiment of the invention, anaerobic bacteria such as those from the genus Clostridium are used to produce ethanol from carbon monoxide, carbon dioxide and hydrogen via the acetyl CoA biochemical pathway. There are a variety of microorganisms that can be used in the fermentation process, and particularly, preferred as an option for use are anaerobic bacteria such as *Clostridium Ijungdahlii* strain PETC or ERI2, which can be used to produce ethanol.

Regarding hydrocarbon intermediates, the Fischer-Tropsch is one process that may be used to chemically transform carbon dioxide and carbon monoxide into liquid hydrocarbons. When doing so, carbon dioxide feedstocks are first converted to carbon monoxide by a reverse water gas shift reaction.

An alternate method for transformation into hydrocarbon intermediates includes solar photothermochemical alkane reverse combustion reactions. These reactions are a one-step conversion of carbon dioxide and water into oxygen and hydrocarbons using a photothermochemical flow reactor.

New processes, like those at Stanford University, have been discovered, using a single step to remove oxygen from carbon dioxide and add hydrogen in the presence of a catalyst having ruthenium and iron oxide nanoparticles.

Additional examples of carbon capture technologies suitable to generate ethanol for use in manufacturing, for example, ethoxy sub-units for use in the surfactants and ethoxylated ingredients described herein (i.e., suitable for fast moving consumer goods) are disclosed in WO 2007/117157, WO 2018/175481, WO 2019/157519 and WO 2018/231948.

The wash composition of the present invention can comprise anionic surfactant, nonionic surfactant or both that includes carbon obtained from carbon capture. The anionic surfactant is from 10 to 100% by weight alkyl ether sulfate, an acyl isethionate or a mixture thereof based on total weight of anionic surfactant. The nonionic surfactant is from 10 to 100% alcohol ethoxylate based on total weight of nonionic surfactant. In an embodiment of the invention, at least 25 to 100%, and preferably, from 40 to 100%, and most preferably, from 50 to 100% or even from 25 to 45% or 65 to 90% (or 10 to 25% or 70 to 100% or 85 to 100% or 92 to 100% or 100%) by weight of the anionic surfactant suitable for use is alkyl ether sulfate. In another embodiment of the invention, at least 25 to 100%, and preferably, from 40 to 100%, and most preferably, from 50 to 100% or even from 25 to 45% or 65 to 90% (or 10 to 25% or 70 to 100% or 85 to 100% or 92 to 100% or 100%) by weight of the anionic surfactant selected for use is an acyl isethionate. When both an alkyl ether sulfate and an acyl isethionate are used as anionic surfactant, the weight ratio of alkyl ether sulfate to acyl isethionate is from 1:10 to 10:1, and preferably, from 1:5 to 5:1, and most preferably, from 1:3 to 3:1 (or from 1:2 to 2:1 or 1:1.5 to 1.5:1 or 1:1). The nonionic surfactant is from 10 to 100% alcohol ethoxylate based on total weight of nonionic surfactant. In an embodiment of the invention, 25 to 100%, and preferably, from 40 to 100%, and most preferably, from 50 to 100% or even from 25 to 45% or 65 to 90% (or 70 to 100% or 85 to 100% or 92 to 100% or 100%) by weight of the nonionic surfactant selected for use is alcohol ethoxylate. Again, at least 10% by weight of the anionic surfactant and/or the nonionic surfactant used comprises carbon obtained from carbon capture. In an embodiment of the invention, from 15 to 100%, and preferably, from 25 to 80%, and most preferably, from 30 to 65% (or 30 to 45% or 30 to 40% or 32 to 38% or 100% by weight of total surfactant) of the anionic and/or nonionic surfactant comprises carbon obtained from carbon capture. In yet another embodiment of the invention, one anionic surfactant used comprises carbon from carbon capture. In another embodiment, both anionic surfactants (alkyl ether sulfate, an acyl isethionate) used comprise carbon from carbon capture. In still another embodiment of the invention, when alkyl ether sulfate, an acyl isethionate and/or a mixture thereof are used with alcohol ethoxylate, two or all three of the components will comprise carbon from carbon capture.

The carbon derived from carbon capture may be found anywhere within the chemical structure of the surfactant or optional ethoxylated ingredient used. The carbon derived from carbon capture can form part of an alkyl chain or an ethoxylate group, and preferably, is provided in an ethoxylate group.

As to the anionic and nonionic surfactants described and the optional ethoxylated ingredient (like polyether) that do contain carbon from carbon capture in an ethoxylate portion, at least 10% by weight of the carbon atoms in such surfactant in the ethoxylate portion are obtained from carbon capture, preferably, at least 15%, and more preferably, at least 30% by weight, and most preferably, at least 40% by weight (or at least 60% or at least 70% or from 10 to 50% or from 15 to 40% or from 18 to 37% or 100%) of all of the carbon atoms in the ethoxylate portion of the same may be obtained from carbon capture based on all carbons in the ethoxylate portion of such surfactants and optional ethoxylated ingredients. In yet another embodiment of the invention, less than 90% by weight, and preferably, less than 70% by weight, and more preferably, less than 30% by weight of the carbon atoms in the ethoxylate portion or groups within the surfactants and optional ethoxylated ingredient are obtained directly from virgin fossil fuels.

Where the carbon derived from carbon capture is located in an alkyl chain of the anionic and nonionic surfactants described and the optional ethoxylated ingredient (like polyether), it is within the scope of the invention for at least 0.5% or at least 5% or at least 10% or at least 20% or at least 32% by weight of the carbons in the alkyl chain to be derived from carbon capture, more preferably at least 45% by weight, and most preferably, at least 65% by weight or all (100% by weight) of the carbons in the alkyl chain to be derived from carbon capture. In still another embodiment of the invention, carbon from carbon capture can appear only in the alkyl chain, only in the ethoxylated portion or in both the alkyl chain and ethoxylated portion of the surfactants and the optional ethoxylated ingredients like polyethers described herein. In even another embodiment, from 1 to 50% or from 1 to 15% or from 2 to 8% of all carbons in the alkyl chains can be carbons from carbon capture.

As noted, suitable carbon chains can be obtained from a Fischer-Tropsh reaction. The feedstock for the Fischer-Tropsch may be 100% carbon obtained from carbon capture or may be a mixture of carbon from different sources. For example, carbon gases from natural gas could be used as an option if desired.

As it relates to alkyl chains in the ingredients described that are suitable for using carbon from carbon capture, still yet another option is for any alkyl chain to comprise less than 50%, and preferably, less than 40%, and most preferably, less than 30%, and even more preferably, less than 15% by weight carbon obtained directly from virgin fossil fuels. It is further within the scope of the invention for all carbons in alkyl chains to comprise no carbon obtained directly from virgin fossil fuels or none of the carbons in alkyl chains to include carbon obtained from carbon capture whereby, as noted, only carbon present in ethoxylate groups is recovered from carbon capture.

In an embodiment of the invention, the alkyl chain may be a combination of alkyl groups from carbon capture and alkyl groups from triglycerides, and preferably, triglycerides obtained from plants, such as palm, rice, rice bran, sunflower, coconut, rapeseed, maze, soy, cottonseed, olive oil, or the like. In yet another embodiment of the invention, less than 50%, and preferably, less than 35%, and most preferably, less than 15% (or from 0.01 to 7% or 0.0% (none)) by weight of the alkyl groups are recovered from palm oil or palm kernel oil or both.

Alternate sources of carbon suitable for use include plant-based carbon, for example, ethanol obtained from the fermentation of sugar and starch (i.e., 'bio' ethanol).

To produce ethoxylates from carbon capture, first ethanol, recovered as described, is dehydrated to ethylene via art recognized techniques like those that employ aluminum oxide and zeolite catalysts). The ethylene is then oxidized to form ethylene oxide.

Depending on the desired end use material, many art recognized options are available.

If an alkyl ether sulphate is desired, the corresponding alcohol ethoxylate can be sulphonated to create the anionic surfactant.

If isethionate is desired, ethylene oxide and a bisulfite salt (like a sodium salt) are used to yield, for example, sodium isethionate. The salt is then esterified with a fatty acid to produce the desired anionic surfactant. Processes for making isethionates are known and an illustrative process is described in U.S. Patent No. 5,646,320.

If an alcohol ethoxylate is desired, i.e., nonionic surfactant as herein described, the ethylene oxide can be reacted with a fatty alcohol via a polymerization type reaction. This process is commonly referred to as ethoxylation and gives rise to alcohol ethoxylates. In an embodiment of the invention, the hydrophobic chain of the fatty alcohol comprises carbon from carbon capture and from a plant source. In yet another embodiment, the hydrophobic chain of the fatty alcohol comprises only carbon from a plant source. In still another embodiment, the fatty alcohol comprises only carbon from carbon capture. Plant sources may further be used to make any of the surfactants and polyethers suitable to comprise carbon obtained from carbon capture as defined herein.

If an optional ingredient like a polyethylene glycol is desired, the ethylene oxide can be polymerized, for example in the presence of water and a catalyst to yield a polyethylene glycol chain.

The anionic surfactants classified as alkyl ether sulphates have the formula:

(R-(OR')_{q}-O-SO₃⁻)ₓQ^{x+},

wherein:
R is an alkyl chain. When the ingredient comprising at least one ethoxylate unit and at least one carbon derived from carbon capture is an alkyl ether sulphate, the carbon obtained from carbon capture may be within the alkyl chain or the ethoxylate group. In an embodiment of the invention, the ethoxylate group or both the alkyl chain and ethoxylate group comprise carbon obtained from carbon capture.
R is a saturated or unsaturated C₈-C₁₈ alkyl group, or a C₁₂-C₁₆ alkyl group or R is a saturated C₈-C₁₆, or a saturated C₁₂-C₁₄ alkyl group;
R' is ethylene;
q is from 1 to 18, preferably from 1 to 15, more preferably, from 1 to 10, still more preferably from 1 to 7, or 1 to 5, or 1 or 2;
x is equal to 1 or 2;
Q^{x+} is a suitable cation which provides charge neutrality, preferably sodium, calcium, potassium, or magnesium, more preferably, a sodium cation.

The alkyl ether sulfate preferred for use is sodium lauryl ether sulfate, sodium pareth ether sulfate, alkyl glyceryl ether sulfates or mixtures thereof. Sodium lauryl ether sulfate is often preferred. Sodium stearyl ether sulfate and sodium palmityl ether sulfate can also an option for use,

As to the isethionates suitable for use, they are generally classified as C₈-C₁₈ acyl isethionates. The isethionate surfactants are prepared as noted. After securing alkali metal isethionate, the alkali metal isethionate can be mixed with aliphatic fatty acids preferably having from 6 to 18 carbon atoms and an iodine value of less than 20. At least 75% of the mixed fatty acids may have from 12 to 18 carbon atoms and up to 25% have from 6 to 10 carbon atoms.

Acyl isethionates suitable for use may also include alkoxylated isethionates such as those described in Ilardi et al., U.S. Pat. No. 5,393,466, entitled "Fatty Acid Esters of Polyalkoxylated isethonic acid; issued Feb. 28, 1995; incorporated herein by reference and previously noted herein. Such surfactants have the general formula:

R¹C--O(O)--C(X)H--C(Y)H₂--(OCH-CH₂)ₘ--SO₃M

wherein R¹ is an alkyl group having 8 to 18 carbons, m is an integer from 1 to 4, X and Y are each independently hydrogen or an alkyl group having 1 to 4 carbons and M is a solubilizing cation as previously described. In a preferred embodiment of the present invention, the isethionate suitable for use and prepared with carbon derived from carbon capture is lauroyl isethionate, cocoyl isethionate, lauroyl methyl isethionate or a mixture thereof whereby such isethionates are solubilized with a cation which is typically sodium, potassium, ammonium or substituted ammonium. In an embodiment of the invention, at least 75% by weight of the total weight of isethionate that can be used in the invention is sodium lauroyl isethionate. In yet another embodiment, at least 85% by weight, and preferably, at least 95% by weight of the total weight of isethionate that can be used in the wash composition of the invention is sodium lauroyl isethionate. In still another embodiment of the invention, all (100%) of the isethionate suitable to use in the present invention is sodium lauroyl isethionate.

The alcohol ethoxylates suitable for use have the general formula:

R²-Z-(C₂H₄O)ₜ-CH₂-CH₂-OH

wherein R² is an alkyl chain. When the ingredient comprising at least one ethoxylate unit and at least one carbon derived from carbon capture is an alcohol ethoxylate, the carbon obtained from carbon capture may be located in the alky chain or the ethoxylate group. Preferably the ethoxylate or both the alkyl chain and ethoxylate comprise carbon obtained from carbon capture.

R² is preferably a linear alkyl with 8 to 60, more preferably 10 to 25, even more preferably 12 to 20 and most preferably 16-18 carbons in the chain.

Z is selected from:

-O-, -C(O)O-, -C(O)N(R²)-

or

-C(O)N(R²)R²-

-and is preferably -O-
t is preferably 2 to 100, more preferably 3 to 50, even more preferably 4 to 30, still even more preferably 4 to 10, and most preferably 5 to 7, calculated as a molar average.

Particularly preferably R² is 16-18 and t is 5-7.

These nonionic surfactants can be used in the wash compositions of the invention as effective wetting, spreading, emulsifying and foaming agents.

As to the optional polyethers suitable for use and comprising carbon from carbon capture, the materials, i.e., polyethylene glycols (PEGs), have the general formula: 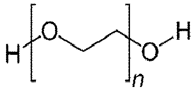 where n is preferably 2 to 200, more preferably 2 to 100, even more preferably 2 to 40, 2 to 30 and most preferably 2 to 20.

The weight average molecular weight of the PEG is preferably 100 to 1300, more preferably, 100 to 1000, most preferably, 100 to 800 or 100 to 600.

The PEG may solely comprise carbon from carbon capture or may comprise carbon from carbon capture in combination with carbon from other sources, as described above, like vegetable sources.

The ethylene oxide and/or polyethylene glycols consistent with this invention can be reacted with ingredients like castor oil to produce PEG ethoxylated castor oil. Preferably the castor oil is ethoxylated with 10 to 80 moles of ethylene oxide, preferably 20 to 60 moles of ethylene oxide. A particularly preferable ingredient is PEG 40 ethoxylated castor oil comprising carbon derived from carbon capture as noted herein.

Percent modern carbon (pMC) level is based on measuring the level of radiocarbon (C¹⁴) which is generated in the upper atmosphere from where it diffuses, providing a general background level in the air. The level of C¹⁴, once captured (e.g., by biomass) decreases over time, in such a way that the amount of C¹⁴ is essentially depleted after 45,000 years. Hence the C¹⁴ level of fossil-based carbons, as used in the conventional petrochemical industry is virtually zero.

A pMC value of 100% biobased or biogenic carbon would indicate that 100% of the carbon came from plants or animal by-products (biomass) living in the natural environment (or as captured from the air) and a value of 0% would mean that all of the carbon was derived from petrochemicals, coal and other fossil sources. A value between 0-100% would indicate a mixture. The higher the value, the greater the proportion of naturally sourced components in the material, even though this may include carbon captured from the air.

The pMC level can be determined using the percent Biobased Carbon Content ASTM D6866-20 Method B, using a National Institute of Standards and Technology (NIST) modern reference standard (SRM 4990C). Such measurements are known in the art are performed commercially, such as by Beta Analytic Inc. (USA). The technique to measure the C¹⁴ carbon level is known and primarily from carbon-dating archaeological organic findings.

In one embodiment, the component (e.g., surfactant) comprising at least one ethoxylate unit and/or at least one carbon derived from carbon capture in an alkyl chain can comprise carbon from point source carbon capture. These will preferably have a pMC of 0 to 10%.

In an alternate embodiment, the component (e.g., surfactant) comprising at least one ethoxylate unit and/or at least one carbon derived from carbon capture in an alkyl chain can comprise carbon from direct air capture. These preferably have a pMC of 90 to 100%.

In an embodiment of the invention the total amount of surfactant present in the wash composition is over 2.5% by weight to typically 38% by weight (wash composition bars can have up to 52% by weight surfactant), and preferably, from 3.5% to 35%, and most preferably, from 3.75% to 30% or from 4 to 25% or from 4.2 to 20% or from 4.25 to 17.5% by weight of the wash composition. In another embodiment of the invention, the weight ratio of zwitterionic surfactant to anionic surfactant is from 1:8 to 1:2 or from 1:6 to 1:2 or from 1:5 to 1:3 or from 1:4 to 1:1. In another embodiment, the isethionate having carbon from carbon capture is at least 50% by weight of a lauroyl isethionate and the composition comprises less than 11.0% by weight sulfate-based surfactant. In still another embodiment of the invention, wash composition can comprise from 1.1 to 2.2, and preferably, from 1.15 to 2, and most preferably from 1.2 to 1.75 times more zwitterionic surfactant than anionic surfactant. In still another embodiment of the invention, anionic surfactant makes up from 3 to 17%, and preferably from 3.2 to 12%, and most preferably, from 3.25 to 8% by weight of the wash composition. In even another embodiment of the invention, zwitterionic surfactant makes up from 2.5 to 22% by weight of the wash composition, or from 2.75 to 18% or from 3 to 15% by weight of the wash composition or even from 3.25 to 8.25% by weight of the composition.

As to suitable naturally derived preservative that may be used in the wash composition of the present invention, the same may comprises benzoic acid and/or a derivative thereof. In an embodiment of the invention, the naturally derived preservative, when used, is from 50 to 100% by weight benzoic acid, a benzoic acid derivative or mixture thereof based on total weight of the preservative used in the wash composition ready for consumer use. In an optional embodiment of the invention, an additional naturally derived preservative may be used with the benzoic acid and/or derivative thereof. The only limitation with respect to the additional naturally derived preservative that may be used is that the same is suitable for use in a composition that is being used by consumers, and especially used, for topical application. Illustrative examples of the additional yet optional naturally derived preservative that may be used in the present invention includes gluconolactone, calcium gluconate, glucuronic acid lactone, thymol, magnolia extract, biopein, suprapein, neopein, sorbic acid, citric acid, rosemary extract, potassium sorbate mixtures thereof or the like. In a preferred embodiment, the preservative used in the present invention when the pH of the wash composition is under 6.2 is 70 to 100% by weight benzoic acid, a benzoic acid derivative or a mixture thereof based on total weight of the preservative used. In a most preferred embodiment, the preservative used in the present invention is 95 to 100% by weight benzoic acid, benzoic acid derivative or mixture thereof based on total weight of the preservative used when the pH of the wash composition is under 5.5. In still another embodiment of the invention, the preservative used in the present invention is all (100% by weight) benzoic acid, benzoic acid derivative or a mixture thereof based on total weight of preservative used when the pH of the wash composition is under 5.35. In still yet another preferred embodiment, the preservative used in the present invention (i.e., benzoic acid derivative) is sodium benzoate, potassium benzoate, amino benzoate or a mixture thereof. When used, the preferred amino benzoate employed in the composition of the present invention is sodium or potassium p-amino benzoate or a mixture thereof. In yet another preferred embodiment, the preservative used in the present invention is naturally recovered and sodium benzoate.

Regarding the amount of preservative used in the present invention, typically the same makes up from 0.20 to 2.0%, and preferably, from 0.3 to 1.5%, and most preferably, from 0.35 to 1.5% by weight of the composition. In still another embodiment, the naturally derived preservative makes up from 0.4 to 0.65% by weight of the composition.

An additional anionic surfactant suitable for optional use, in addition to alkyl ether sulfate and/or acyl isethionate, in the present invention is an alkyl sulfate. The alkyl sulfate (e.g., C₁₂-C₁₈ alkyl sulfate) suitable for use includes sodium, potassium, ammonium or substituted ammonium salts of lauryl sulfate, pareth sulfate, myreth sulfate, stearyl sulfate, capric sulfate or a mixture thereof.

Regarding the alkyl ether sulfate-based surfactant used in this invention with carbon from carbon capture, such preferred alkyl ether sulfate, sodium lauryl ether sulfate, is suitable for use with the preferred alkyl sulfate, sodium lauryl sulfate. In an embodiment of the invention, the sulfate-based surfactant used is at least 35%, and preferably, at least 40%, and most preferably, from 45 to 95% by weight sodium lauryl ether sulfate, based on total weight of sodium lauryl ether sulfate and sodium lauryl sulfate that make up the sulfate-based surfactant suitable for use. Again, in an embodiment of the invention, such sodium lauryl ether sulfate used in the invention can have 1, 2, 3, 4, 5, 6 or 7 polyethylene oxide units (e.g., EO units) or a mixture thereof. In an often-preferred embodiment, the sodium lauryl ether sulfate used is a mixture of surfactants with 1, 2 and 3 EO units. In still another preferred embodiment, any mixture of sulfate-based surfactant will have between 20 to 90%, and preferably 45 to 65% by weight sodium lauryl ether sulfate and from 10 to 80%, and preferably, 35 to 55% by weight sodium lauryl sulfate, based on total amount of sulfate-based surfactant in the wash composition. It is within the scope of the invention for the sulfate-based surfactant to consist only of sodium lauryl ether sulfate.

In an especially preferred embodiment, the alkyl ether sulfate used comprises an alkyl portion having from 25 to 100%, and preferably, from 35 to 90%, and most preferably, from 50 to 85% by weight C₁₆-C₁₈ alkyl chains based on total weight of alkyl chains and an ether portion (ethylene oxide/EO) having from 25 to 100%, and preferably, from 35 to 90%, and most preferably, from 50 to 85% by weight ether portion with 4 to 7 or 4 to 6 or 4 to 5 ethylene oxide (EO) groups based on the total weight of ether portion.

Other optional anionic surfactants suitable for use include those classified generally as taurates. Illustrative taurates suitable for use include acyl taurates, including methyl acyl taurates. Often, the taurates that may be used in the present invention include those generally identified by the formula:

R³CONR⁴CH₂CH₂SO₃M

wherein R³ is a C₈-C₂₀ alkyl, R⁴ is a C₁-C₄ alkyl.

M is a solubilizing cation as previously described.

In an embodiment of the invention, when a taurate is used sodium methyl lauroyl taurate, sodium methyl cocoyl taurate or a mixture thereof is generally preferred. In another embodiment of the invention, it is suitable to include carbon derived from carbon capture in amounts described for anionic surfactants noted herein.

As to glycinates suitable for use as additional anionic surfactants, these include acyl glycinates like sodium lauroyl glycinate, sodium cocoyl glycinate or a mixture thereof.

In still another embodiment of the invention, anionic surfactant can make up from 4 to 15%, and preferably, from 5 to 12%, and most preferably, from 6 to 10% by weight of the wash composition. In an often-desired embodiment, sulfate-based surfactant makes up at least 4.0% by weight of the total weight of the composition but less than 11% by weight of the wash composition. In still another embodiment of the invention, isethionate which is preferably at least 50% by weight sodium lauroyl isethionate based on total amount of isethionate used in the composition, typically makes up from 0.28 to 4.6% or 0.4 to 4%, and preferably, from 0.5 to 2%, and most preferably, from 0.75 to 1.5% by weight of the composition.

As to the zwitterionic surfactant used in the present invention, the same typically comprises a betaine, sultaine or mixture thereof, including simple betaines of formula:

R⁵--N⁺--(R⁶)(R⁷)CH₂CO₂⁻

and amido betaines of formula:

R⁵--CONH(CH₂)ᵣ--N⁺--(R⁶)(R⁷)CH₂CO₂-

where r is 2 or 3.

In both formulae, independently, R⁵ is alkyl or alkenyl of 7 to 18 carbon atoms; R⁶ and R⁷ are each independently alkyl, hydroxyalkyl or carboxyalkyl of 1 to 3 carbon atoms; R⁵ may, in particular, be a mixture of C₁₂ and C¹⁴ alkyl groups derived from coconut oil so that at least half, preferably at least three quarters of the groups R⁵ have 10 to 14 carbon atoms. R⁶ and R⁷ are preferably methyl.

A further possibility is that the zwitterionic surfactant is a sulphobetaine of the formula:

R⁵--N⁺--(R⁶)(R⁷)(CH₂)₃SO₃⁻

or

R⁵--CONH(CH₂)ᵤ --N⁺--(R⁶)(R⁷)(CH₂)₃SO₃⁻

where u is 2 or 3, or variants of these in which --(CH₂)₃SO₃⁻ is replaced by-CH₂C(OH)(H)CH₂SO₃⁻.

In these formulae, R⁵, R⁶ and R⁷ are as previously defined.

Illustrative examples of the zwitterionic surfactants suitable that may be used include betaines like cocodimethyl carboxymethyl betaine, cocoamidopropyl betaine, laurylamidopropyl betaine or mixtures thereof. An additional zwitterionic surfactant suitable for use includes cocoamidopropyl sultaine. In one embodiment of the invention, the zwitterionic surfactant used is a betaine and at least 65% by weight, and preferably, at least 80% by weight, and most preferably, at least 95% by weight cocoamidopropyl betaine based on total weight of zwitterionic surfactant used. In an especially preferred embodiment of the invention, the zwitterionic surfactant used is all cocoamidopropyl betaine. Such zwitterionic surfactants are made commercially available from suppliers like Stepan Company.

In one embodiment of the invention the betaine (i.e., zwitterionic surfactant) used typically makes up from 0.8 to 15%, and preferably, from 1.0 to 7%, or 1.0 to 6.0%, and most preferably, from 1.5 to 3.5% by weight of the composition. In even another preferred embodiment, the zwitterionic surfactant is a betaine and makes up from 2 to 3% by weight of the wash composition.

The polyol suitable for use in the present invention is limited only to the extent that it is suitable for use in a topical composition and water soluble. Illustrative and nonlimiting examples of the polyols that may be used in the present invention include sorbitol, glycerol, mannitol, xylitol, maltitol or mixtures thereof. In an embodiment of the invention, the polyol used is at least 50% by weight glycerol, based on total weight of the polyol used in the composition. In another embodiment of the invention, the polyol used is all glycerol (100% by weight). Polyol will typically make up from 0 to 55%, or from 0.01 to 35% or from 1 to 23% or from 1.5 to 9% by weight of the composition, and preferably, from 2 to 8% by weight of the wash composition, and most preferably, from 2 to 7% by weight of the wash composition of the present invention.

The wash composition (when a liquid ready to use) of the present invention will comprise 40 to 90% by weight water, and more preferably, from 45 to 85% by weight water, and most preferably, from 50 to 80% by weight water. Concentrates will typically have 45 to 55% by weight of the water defined for ready to use liquid wash composition. In an embodiment of the invention, products that are anhydrous will have less than 7% or less than 6% or less than 5% or (no 0.0%) by weight water. In another embodiment, anhydrous products will comprise from 0.01 to 3.5% by weight water.

The liquid wash composition is preferably isotropic, and typically translucent or transparent. It is within the scope of the invention for the composition to be lamellar, and if lamellar, the same can include from 0.1 to 12%, and preferably, 0.15 to 10%, and most preferably, 0.2 to 8% by weight of a liquid (i.e., lamellar) structuring agent based on total weight of the liquid composition of the invention.

Such structuring agents are typically fatty acids (or ester derivatives thereof) or fatty alcohols. Such materials include C₈-C₂₂ acids such as the following: caprylic acid, lauric acid, myristic acid, oleic acid, isostearic acid, linoleic acid, linolenic acid, ricinoleic acid, elaidic acid, arachidonic acid, myristoleic acid, palmitoleic acid, mixtures thereof or the like. Ester derivatives include propylene glycol isostearate, propylene glycol oleate, glyceryl isostearate, glyceryl oleate and polyglyceryl diisostearate, mixtures thereof or the like. Regarding the fatty alcohols suitable for use, these include cetyl alcohol, stearyl alcohol, behenyl alcohol, mixtures thereof or the like. In an embodiment of the invention, from 3 to 25% by weight, or from 5 to 15% by weight of the carbon in such structuring agents is carbon obtained from carbon capture.

The structuring agent is preferably a fatty acid. More preferably, the structurant is selected from the group consisting of caprylic acid, lauric acid, myristic acid or a mixture thereof. In an especially preferred embodiment, the structuring agent is lauric acid.

Water activity of the composition of this invention is typically under 1, preferably from 0.94 to 0.99, and most preferably, from 0.95 to 0.98 as measured with an Aqualab Activity Meter. Oils may optionally be used in the wash compositions of the present invention. Oils suitable for use include silicone oils. Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, and preferably, from 4 to 5 silicon atoms. Nonvolatile silicone oils useful in this invention include polyalkyl siloxanes, polyalkylaryl siloxanes and polyether siloxane copolymers. Such essentially non-volatile polyalkyl siloxanes useful herein include, for example, polydimethylsiloxanes (like dimethicone) with viscosities of from 5 to 100,000 centistokes at 25°C. A silicone source that may be used is cyclopentasiloxane and/or a dimethicone solution. In one embodiment of the invention, the wash composition of the present invention comprises no (0.0%) by weight decamethylcyclopentasiloxane (D5).

Suitable esters for optional use in the wash composition include: (1) Alkenyl or alkyl esters of fatty acids having 10 to 20 carbon atoms like isopropyl palmitate, isopropyl isostearate, isononyl isonanonoate, oleyl myristate, isopropyl myristate, oleyl stearate, and oleyl oleate; (2) Ether-esters such as fatty acid esters of ethoxylated fatty alcohols; (3) Polyhydric alcohol esters such as ethylene glycol mono- and di-fatty acid esters, diethylene glycol mono- and di-fatty acid esters, polyethylene glycol (200-6000) mono and di-fatty acid esters, propylene glycol mono- and di-fatty acid esters, polypropylene glycol 2000 monooleate, polypropylene glycol 2000 mono stearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxy-ethylene sorbitan fatty acid esters; (4) Sterol esters, of which soya sterol and cholesterol fatty acid esters are examples thereof.

Still other oils that may be used include triglycerides (animal and/or vegetable) like soybean oil (including hydrogenated soybean oil), sunflower oil, coconut oil, palm kernel oil, castor oil, rapeseed oil, palm oil, grape seed oil, caprylic/capric triglyceride, moringa oil, safflower oil, fish oil or mixtures thereof.

Even other oils suitable for use include mineral oil, jojoba oil, isoparaffins, C₁₂-C₁₅ alkyl benzoates, polyalphaolefins, isohexadecane, petrolatum, mixtures thereof (including with those oils above) or the like. Soybean and sunflower oil are often preferred triglyceride oils.

These optional oils suitable for use make up from 0 to 10% by weight of the wash composition. In an embodiment of the invention, petrolatum makes up from 0.01 to 6%, and preferably, from 0.5 to 5%, and most preferably, from 1.0 to 4.0% by weight of the composition.

In an embodiment of the invention, wash compositions and especially those that are shampoos or shampoos and conditioners comprise both shea butter and almond kernel oil and often, individually, at amounts from 0.01 to 1.3% or from 0.02 to 1 % by weight of the wash composition.

Adjusters suitable to modify the pH of the compositions of this invention may be used. Such pH adjusters include triethylamine, NaOH, KOH, H₂SO4, HCl, C₆H₈O₇ (i.e., citric acid) or mixtures thereof. The pH adjusters are added at amounts such that the final pH of the composition is as defined herein.

The pH of the composition is assessed by using conventional instrumentation such as a pH meter made commercially available from Thermo Scientific^{®}.

Concentrates described herein are diluted with water, typically from 1:4 to 1:2 concentrate to water, respectively. Bars described herein are diluted with water, typically from 10:1 to 7:1 water to bar, respectively. Anhydrous compositions described herein are diluted with water, typically from 1:5 to 1:2 concentrate to water, respectively.

In an embodiment of the invention, the wash composition of the present invention may include optional surfactants in addition to the anionic, nonionic and zwitterionic surfactants herein described. In an embodiment of the invention, when such surfactants are used in the composition of the present invention, they typically make up no more than 8% by weight of the composition. In an embodiment of the invention, when such additional surfactants are used, they typically make up from 0.0001 to 7% and preferably, from 0.001 to 5% by weight of the composition. Therefore, it is within the scope of the present invention to optionally include conventional soaps and/or syndets in the compositions of the present invention.

Optional surfactants that may be used include alkyl sulfosuccinates (including mono- and dialkyl, e.g., C₆-C₂₂ sulfosuccinates); alkyl and acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides, and the like.

In an embodiment of the invention, additional optional surfactants can include sodium lauroyl glutamate, sodium cocoyl glutamate or a mixture thereof. Such anionic surfactants described herein are commercially available from suppliers like Galaxy Surfactants, Clariant, Sino Lion and Innospec.

Amphoteric surfactants that optionally may be used in the compositions (which depending on pH can be zwitterionic) of the present invention include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for optional use include sodium lauroamphoacetate, sodium cocoamphoacetate, sodium lauroamphoacetate, sodium cocoamphoacetate or mixtures thereof.

Nonionic surfactants suitable for optional use include long chain tertiary amine oxides, long chain tertiary phosphine oxides, dialkyl sulphoxides or the like.

The optional nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995 or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

Cationic surfactants may optionally be used in the compositions of the present invention.

One class of cationic surfactants suitable for optional use includes heterocyclic ammonium salts such as cetyl or stearyl pyridinium chloride, alkyl amidoethyl pyrrylinodium methyl sulfate, and lapyrium chloride.

Tetra alkyl ammonium salts are another useful optional class of cationic surfactants suitable for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used optionally are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Even other useful cationic surfactants suitable for optional use include quaternized hydrolysates of silk, wheat, and keratin proteins, and it is within the scope of the invention to use mixtures of the aforementioned cationic surfactants.

Unlike the other optional surfactants suitable for use, if cationic surfactants are used, they will typically make up no more than 1.0% by weight of the composition. If present, they typically make up from 0.001 to 0.7%, and more typically, from 0.001 to 0.5% by weight of the wash composition.

In an embodiment of the invention, only the surfactants which are sulfate-based including carbon derived from carbon capture; isethionates including carbon derived from carbon capture, taurates and/or glycinates; and betaines and/or sultaines are used in the wash composition of the invention. In still another embodiment, alcohol ethoxylate with carbon obtained from carbon capture is used in the wash compositions in addition to the same. In yet another embodiment, the total amount of surfactant used in the composition of the present invention (with or without optional surfactants) is preferably less than 21% by weight of the wash composition. It is, however, within the scope of the invention, if desired, to include carbon obtained from carbon capture in all surfactants used herein.

In an embodiment of this invention, the composition of the present invention is substantially free of (less than 0.25% by weight of the composition) polymeric quaternary ammonium compounds (including salts of the same). In another embodiment, the composition of this invention will comprise less than 0.15% (or less than 0.5% or 0.1 %) by weight polymeric quaternary ammonium compounds. In yet another embodiment, the composition will comprise less than 0.01% by weight polymeric quaternary ammonium compounds. In even another embodiment, the composition is free of polymeric quaternary ammonium compounds (i.e., 0.0%).

Optional skin benefit agents suitable for use in the composition of this invention are limited only to the extent that they are capable of being topically applied, and suitable to dissolve in the wash composition at the desired pH.

Illustrative examples of the benefit agents suitable to include in the water portion of such compositions are acids, like amino acids, such as arginine, valine or histidine. Additional water-soluble benefit agents suitable for use include vitamin B₂, niacinamide (vitamin B₃), vitamin B₆, vitamin C, mixtures thereof or the like. Water soluble derivatives of such vitamins may also be employed. For instance, vitamin C derivatives such as magnesium ascorbyl phosphate and ascorbyl glycoside may be used alone or in combination with each other. Other water-soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water-soluble benefit agents (including mixtures) when present in the composition of the invention may range from 0.0 to 10%, preferably from 0.001 to 8%, and most preferably, from 0.01 to 6% by weight, based on total weight of the wash composition and including all ranges subsumed therein.

In an embodiment of the invention, salicylic acid, benzalkonium chloride, benzethonium chloride, cetrimonium chloride, benzoyl peroxide, adapalene, glycolic acid, lactic acid, Triclosan, chloroxylenol, triclocarban, climbazole, dodecyl dimonium chloride, cetylpyridinium chloride or mixtures thereof are often desired for use when the wash compositions are formulated and marketed to reduce acne. Such actives typically make up from 0.01 to 3%, and preferably, from 0.1 to 2%, and most preferably, from 0.2 to 1.5% by weight of the wash composition.

It is also within the scope of the present invention to optionally include oil (i.e., non-water) soluble benefit agents. The only limitation with respect to such oil soluble benefit agents are that the same are suitable to provide a benefit to skin when topically applied.

Illustrative examples of the types of oil soluble benefit agents that may optionally be used in the wash composition of this invention include components like stearic acid, palmitic acid, vitamins like vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol, thiamidol or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 10-hydroxystearic acid, 12-hydroxystearic acid, petroselinic acid, conjugated linoleic acid, stearic acid, palmitic acid, lauric acid, terpineol, thymol mixtures thereof or the like. Ricinoleic acid is also suitable for use.

In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred. Still another retinoic acid precursor suitable for use is hydroxyanasatil retinoate made commercially available under the name Retextra^{®} as supplied by Molecular Design International. The same may be used in a mixture with the oil soluble actives described herein.

When optional (i.e., 0.0 to 1.5% by weight) oil soluble active is used in the oil (including surfactant) phase of the composition of the invention, it typically makes up from 0.001 to 1.5%, and in another embodiment, from 0.05 to 1.35%, and in yet another embodiment, from 0.1 to 1.2% or 0.1 to 1% or 0.1 to 0.5% by weight of the total weight of the wash composition. In an embodiment of the invention, palmitic acid and/or 12-hydroxystearic acid and glycerol are present in the wash compositions of the invention, with or without niacinamide.

In another embodiment of the invention, from 0.001 to 1.5% or from 0.01 to 1% by weight hyaluronic acid and/or dihydroxyacetone is used in the wash compositions if desired.

It is within the scope of the present invention to optionally include traditional preservatives (in addition to any naturally derived preservatives used in this invention) in the compositions of this invention to protect against the growth of potentially harmful microorganisms. Cosmetic chemists are familiar with appropriate preservatives and routinely choose them to satisfy the preservative challenge test and to provide product stability. Suitable traditional but optional preservatives for use include hydantoin derivatives and propionate salts. Particularly preferred preservatives are iodopropynyl butyl carbamate, phenoxyethanol (with or without 1,2-octanediol), hydroxyacetophenone, ethylhexylglycerine, hexylene glycol, methyl paraben, propyl paraben, imidazolidinyl urea, dimethyl-dimethyl (DMDM) hydantoin and benzyl alcohol and mixtures thereof. Other preservatives suitable for use include chlorophenesin and decylene glycol. The preservatives should be selected having regard for the use of the composition and possible incompatibilities between the preservatives and other ingredients in the composition. These optional preservatives, when used, may be employed in amounts ranging from 0.001 % to 2% by weight of the total weight of the composition, including all ranges subsumed therein. In a preferred embodiment of the invention, only the naturally derived preservative comprising benzoic acid and/or a derivative thereof is used without any additional and optional preservatives. It should also be understood that benzoic acid and/or derivatives thereof that are synthetically made are suitable for use in this invention and at the desired pH values, either with or without the naturally derived preservatives.

As noted herein preservative that includes phenoxyethanol (with or without capryloyl glycine and/or undecylenoyl glycine), iodopropynyl butylcarbamate, benzoic acid (and/or a derivative of benzoic acid natural or synthetic) as well as mixtures thereof are very suitable and often desired for use in the compositions of the invention.

Again, it is preferred that the compositions are free of or substantially free of isothiazolinones, hydantoins and parabens.

As to heterocyclic impurities, like 1,4-dioxane, the same can be removed with biofilters (nitrogen removal biofilters), and processes that employ ozone and ozone with peroxide where the impurities may be removed from solutions comprising them including those with sulfated surfactants. The wash compositions of the present invention can be formulated to comprise less than 25 ppm heterocyclic impurities. Preferably, the wash compositions have less than 10 ppm, and preferably, less than 5 ppm, and most preferably, less than 2 ppm, and less than 1 ppm or even less than 0.05 ppm or no heterocyclic impurities like 1,4-dioxane.

Thickening agents are optionally suitable for use in the wash composition of the present invention. Particularly useful are the polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g., cellulose microfibrils, cellulose nanocrystals or microcrystalline cellulose). Sources of cellulose microfibrils include secondary cell wall materials (e.g., wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers are yet another class of effective thickening agent. This category includes crosslinked polyacrylates such as the Carbomers, acrylate copolymers, acrylates/ acrylate (C₁₀-C₃₀) alkyl acrylate crosspolymers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, salts like sodium chloride, fumed silica, and magnesium-aluminum-silicate may also be used.

The amounts of the thickening agent, when used, may range from 0.001 to 5%, by weight of the composition. Often, thickeners are present at from 0.8 to 3.5% by weight, and preferably, from 1.0 to 3.0% by weight of the wash composition when petrolatum (0.01 to 1% by weight) is included. In an embodiment of the invention, cationic thickeners will often make up from 0.01 to 2.5% and preferably from 0.05 to 1.8%, and most preferably, from 0,2 to 1.2% by weight of the wash composition.

Fragrances, fixatives, chelators (like EDTA) salts (like NaCl) and exfoliants may optionally be included in the wash composition of the present invention. Each of these substances may range from about 0.03 to about 5%, preferably between 0.1 and 3% by weight of the total weight of the composition, including all ranges subsumed therein. To the extent the exfoliants are used, those selected should be of small enough particle size so that they do not impede the performance of any pump and actuator used to dispense composition of this invention.

Salts, especially NaCl, are typically preferred for use to aid in viscosity building, often at 0.5 to 2% and preferably from 0.75 to 1.55% by weight of the wash composition and even from 0.8 to 1.45% by weight of the wash composition. Compositions of this invention comprising surfactants having carbon obtained form carbon capture may surprisingly be thickened in a manner consistent with compositions having conventional surfactants obtained with vegetable and/or petroleum based sources.

In an embodiment of the invention and when used, polyether having carbon obtained from carbon capture will make up from 0.01 to 15%, preferably from 0.03 to 6%, and most preferably, from 0.5 to 4% by weight of the wash composition.

Conventional humectants, in addition to the polyols previously described, may optionally be employed as additives in the present invention to assist in moisturizing skin when the compositions are topically applied. These are generally polyhydric alcohol type materials. Typical polyhydric alcohols (in addition to the polyethylene glycol suitable to include carbon obtained from carbon capture described) include dipropylene glycol, polypropylene glycol (e.g., PPG-9), sorbitol, hydroxypropyl sorbitol, hexylene glycol, 1,3-butylene glycol, isoprene glycol, 1,2,6-hexanetriol, propoxylated glycerol and mixtures thereof. When used, the amount of optional humectant employed may range anywhere from 0.0 to 15% by weight of the total weight of the composition. Often, optional humectant makes up from 0.0 to 10%, and preferably, from 0.001 to 8% by weight (most preferably, from to 3% by weight) of the total weight of the wash composition.

Another optional additive suitable for use includes hemp oil with 2.5 to 25% by weight cannabigerol and/or cannabidiol at from 0.5 to 10 percent by weight. When used, such oil makes up from 0.0001 to 12% by weight of the composition, and preferably, from 0.01 to 5% by weight of the wash composition.

When wash composition in the form of a bar is desired, the surfactants and polyethers having the carbon from carbon capture may be used along with C₁₄ to C₁₈ fatty acids, and preferably, 18 to 41%, and preferably, 19 to 40%, and most preferably, 20 to 38% by weight of such fatty acids are used where stearic acid is especially preferred. Such desired bars will also likely have C₈ to C₁₄ fatty acids and typically from 2 to 9%, and preferably, 3 to 8%, and most preferably, 4 to 7% by weight of such fatty acids where lauric acid is especially preferred. The amount of water in the bars will be less than 9% by weight, preferably, less than 7% by weight water, and most preferably, the bar will comprise from 1 to 6% or 1 to 5% or 1 to 4% by weight water based on total weight of the bar. Total surfactant in the bar is typically 38% (optionally up to 52% by weight) by weight or less, and preferably, from 25 to 32% by weight or 19 to 27% by weight. In one embodiment of the invention, antibacterial agents, like salicylic acid are preferred for use. In another embodiment, salicylic acid makes up 0.01 to 5%, and preferably, at from 0.5 to 3% by weight of the bar composition to deliver anti-acne benefits when used.

When the wash composition of the present invention is a shampoo or shampoo and conditioner, the compositions may comprise conventional silicone emulsions and preferably do comprise cationic polymers selected from cationic cellulose and cationic guar derivatives. Particularly preferred cationic polymers are JAGUAR C13S, JAGUAR C15, JAGUAR C16 JAGUAR C17, JAGUAR C162 or mixtures thereof. In an embodiment of the invention, such polymers make up from 0.2 to 1.2% by weight of the composition.

As to end use compositions that are shampoo compositions or shampoos and conditioners, these are suitable to comprise the surfactants and polyethers having the carbon from carbon capture as described herein. Such shampoo compositions may further comprise anti-dandruff agents like climbazole, picotone olamine, zinc pyrithione, zinc sulfite, selenium sulphide or mixtures thereof. Anti-dandruff agents will usually make up from 0.1 to 5% by weight of the shampoo composition.

Additional optional but often preferred additives suitable for use in shampoos and shampoos and conditioners include acrylic based pressure sensitive adhesives (like n-butyl acrylate), silicone conditioning agents, haircare adjuvants (e.g., amino acids, hair root nutrients and styling polymers). Other optional additives include agents like sweet almond, fruit extract, biotin, gluconolactone, trehalose, cocamide MEA, collagen amino acids, hydrolyzed wheat protein, hydrolyzed elastin, hydrolyzed keratin, behentrimonium chloride, argenia spinosa keratin oil, limonene, hexyl cinnamal, amododimethicone, mica, titanium dioxide, linalool, mixtures thereof or the like. When used, each of such additives will make up less than 4% by weight of the wash composition and preferably from 0.001 to 2.5%, and most preferably, from 0.001 to 1.5% or from 0.01 to 1% by weight of the wash composition. Thickening agent like polyquaternium 10 sold, for example, as Polymer BHA-10 from Dow. In an embodiment of the invention and when used, the same may make up 0.001 to 1% by weight of the wash composition.

When making the composition of the present invention, the desired ingredients may be mixed using conventional apparatus under moderate shear and atmospheric conditions, with temperature being from 25 to 90°C, and preferably, from 30 to 80°C. A homogeneous emulsion is typically recovered as wash composition consistent with the invention and with components comprising carbon obtained form carbon capture. If desired, conventional homogenization with a sonolator (like those commercially supplied from Sonic Corporation).

Unexpectedly, it has been discovered that inclusion of ingredient comprising at least one ethoxylate unit and/or at least one carbon derived from carbon capture allows for improved consumer satisfaction in terms of fragrance, stability, sensory and/or cleaning performance.

It was also surprisingly found that inclusion of ingredient with carbon obtained from carbon capture not only improves the ecological profile of the product but such ingredient does not negatively influence the performance or other characteristics of the product, like stability (including viscosity), sensory and fragrance characteristics, whereby headspace fragrance in packaging may be improved.

The packaging for the wash composition of this invention is limited only to the extent that it is desired for use by consumers and suitable to carry the wash composition of the invention. Typically, the packaging can be a bottle, jar, tube, biodegradable sachet or tub. Preferably, the packaging is durable yet light weight and comprises at least 10% by weight or more of post-consumer resin (PCR) to ensure that its impact on the environment is limited. When concentrates or anhydrous products are desired, refillable packaging is suitable for use. In a preferred embodiment, the preservative system of the invention is natural, and/or the packaging is all PCR or biodegradable and the anionic and nonionic surfactants used have carbon derived from carbon capture. In another preferred embodiment, less than 15%, and preferably less than 10%, and most preferably, less than 5% (or less than 2% or less than 1% or 0.0% (none)) of the surfactants and/or structurants used comprise hydrophobic groups derived from palm kernel oil.

Other embodiments of the claimed wash composition of the invention include:
The wash composition according to the invention, wherein the wash composition is a liquid hand wash, face wash, shampoo or shampoo and conditioner.
The wash composition according to the invention, wherein the composition is a bar, anhydrous powder or concentrate.

The wash composition according to the invention, wherein the composition is a shampoo or shampoo and conditioner further comprising shea butter, almond kernel oil, sweet almond, fruit extract, biotin, gluconolactone, trehalose, cocamide MEA, collagen, amino acid, hydrolyzed wheat protein, hydrolyzed elastin, hydrolyzed keratin, behentrimonium chloride, argenia spinosa keratin oil, limonene, hexyl cinnamal, amododimethicone, mica, titanium dioxide, linalool, polyquaternium 10 or mixtures thereof.

20. The wash composition according to the invention, wherein the composition further comprises petrolatum, sodium benzoate or both.

The wash composition according to the invention, wherein the composition further comprises salicylic acid, benzalkonium chloride, benzethonium chloride, cetrimonium chloride, benzoyl peroxide, adapalene, glycolic acid, lactic acid, Triclosan, chloroxylenol, triclocarban, climbazole, dodecyl dimonium chloride, cetylpyridinium chloride or mixtures thereof.

The wash composition according to the invention, wherein the composition further comprises climbazole, picotone olamine, zinc pyrithione, zinc sulfite, selenium sulphide or mixtures thereof.

The wash composition according to the invention, further comprising thiamidol, niacinamide, 12-hydroxystearic acid, glycerol, palmitic acid or a mixture thereof.

The wash composition according to the invention, wherein the composition is an anti-dandruff shampoo.

25. The wash composition according to the invention, wherein the composition is a shampoo or shampoo and conditioner further comprising acrylic-based pressure sensitive adhesive.

The wash composition according to the invention, wherein the composition is a shampoo, conditioner and body wash.

The wash composition according to the invention, wherein the composition is substantially free of isothiazolinones, hydantoins and parabens.

The wash composition according to the invention, wherein the composition comprises less than 25 ppm heterocyclic impurities. Preferably, wherein the heterocyclic impurity is 1,4-dioxane.

A wash composition according to the invention, wherein the composition further comprises a taurate with and/or without carbon obtained from carbon capture.

The wash composition according to the invention, wherein the composition is a concentrate, anhydrous powder, liquid, bar, or impregnated on a substrate.

The wash composition according to the invention, wherein the composition is liquid having a viscosity from 750 to 22,000 cps.

The wash composition according to the invention, wherein the composition is packaged in a post-consumer resin package.

The wash composition according to the invention, wherein the composition comprises terpineol and thymol.

The wash composition according to the invention, wherein the wash composition is a face wash.

The wash composition according to the invention, further comprising phenoxyethanol, phenoxyethanol and capryloyl glycine and/or undecylenoyl glycine, iodopropynyl butylcarbamate, benzoic acid a derivative of benzoic acid or a mixture thereof.

The wash composition according to the invention, wherein the anionic surfactant comprises further sodium lauroyl isethionate and the composition comprises less than 11.0% by weight sulfate-based surfactant.

The wash composition according to the invention, wherein the composition comprises from 1.1 to 2.2, and preferably, from 1.15 to 2, and most preferably from 1.2 to 1.75 times more 1.2 zwitterionic surfactant than anionic surfactant.

The wash composition according to the invention, wherein the composition further comprises stearic acid and/or palmitic acid and glycerol, the composition optionally comprising thiamidol, ricinoleic acid, petroselinic acid, conjugated linoleic acid, omega-3 fatty acid or a mixture thereof.

The wash composition according to the invention, wherein the anionic surfactant comprises sodium lauryl sulfate and the zwitterionic sulfate comprises a betaine.

The wash composition according to the invention, wherein the composition comprises less than 9% by weight sulfate-based surfactant.

The wash composition according to the invention, further comprising sodium benzoate, potassium benzoate, amino benzoate or a mixture thereof.

The wash composition according to the invention, further comprising an alkyl sulfate, acyl taurate of both.

The wash composition according to the invention, wherein the acyl taurate comprises carbon obtained from carbon capture.

The wash composition according to the invention, wherein the anionic surfactant comprises sodium lauryl ether sulfate with 1, 2, 3, 4, 5, 6 or 7 polyethylene oxide units and preferably 1 or 2.

The wash composition according to the invention, wherein the composition is a liquid and lamellar and comprises a structuring agent with from 3 to 25% by weight, and preferably, from 5 to 15% by weight of carbon obtained from carbon capture.

The wash composition according to the invention, wherein the composition is a liquid and is isotropic.

The wash composition according to the invention, wherein less than 15%, and preferably less than 10%, and most preferably, less than 5% or less than 2% or less than 1% or 0.0% of the surfactants and/or structurants used comprise hydrophobic groups derived from palm kernel oil.

The Examples provided are to facilitate an understanding of the invention. They are not intended to limit the scope of the claims.

### EXAMPLES

The ingredients below are illustrative of ingredients comprising at least one ethoxylate unit and/or at least one carbon derived from carbon capture that may be made via methods previously described.

**Table 1: Alkyl ether sulphate**

| | **Ethoxylate (1EO)** | **Alkyl (C12/14)** |
|---|---|---|
| **Comparative A** | Virgin fossil fuel | Virgin fossil fuel |
| **Example 1** | Virgin fossil fuel | Carbon Capture |
| **Example 2** | Carbon Capture | Coconut oil |
| **Example 3** | Carbon Capture | Carbon Capture |

**Table 2: Alcohol ethoxylate**

| | **Ethoxylate (5EO)** | **Alkyl (C12/14)** |
|---|---|---|
| **Comparative B** | Virgin fossil fuel | Virgin fossil fuel |
| **Example 4** | Virgin fossil fuel | Carbon Capture |
| **Example 5** | Carbon Capture | Coconut |
| **Example 6** | Carbon Capture | Carbon Capture |

**Table 3: Polyethylene glycol (molecular weight 400)**

| | **Ethylene oxide** |
|---|---|
| **Comparative C** | 100 % Virgin fossil fuel |
| **Example 7** | 30 % Virgin fossil fuel, 70 % carbon capture |
| **Example 8** | 50 % bioethanol, 50 % carbon capture |
| **Example 9** | 100 % Carbon Capture |

**Table 4: Alcohol ethoxylate that may be made with longer chain alkyl group (i.e., C₁₆-C₁₈)**

| | **Ethoxylate (5EO)** | **Alkyl (C16/18)** |
|---|---|---|
| **Comparative B** | Virgin fossil fuel | Virgin fossil fuel |
| **Example 10** | Virgin fossil fuel | Carbon Capture |
| **Example 11** | Carbon Capture | Coconut |
| **Example 12** | Carbon Capture | Carbon Capture |

Sensorial testing may be performed on nonionic surfactants which are C₁₂-alcohol ethoxylates with an average of 7 EO groups. A C₁₂-alcohol ethoxylate-7 EO group nonionic surfactant according to the invention may be obtained when the EO-polymer moiety is derived from a process which involves gas fermentation to reduce gaseous captured CO₂ to ethanol, wherein the ethanol may further be converted to ethylene and used to make the EO-polymer. The alkyl-chain may be obtained from a bio-source. A C₁₂-alcohol ethoxylate-7 EO group nonionic not according to the invention may also be obtained, also with an alkyl chain derived from a bio-source, but where the EO-polymer chain can be derived from a petrochemical source. The surfactants may be used to make a wash composition. The wash compositions may be tested at 20 degrees Celsius or heated to 40 degrees Celsius. The compositions, which will otherwise contain no added perfumes may be tested via smell test from trained panelists.

It is expected the surfactant according to the invention and in fast moving consumer products will surprisingly provide a more desirable and 'waxy/fatty' odour whereas the petrochemically derived and traditional surfactant deplete of carbon obtained from carbon capture will provided a 'chemical' odour. The odour perception may be verified by trained panellists independently.

### Example 13

The surfactants and polyethylene glycol identified may be used in wash formulations as described. An illustrative composition that may include any of the surfactants above, and/or polyethylene glycol is as follows:

| Ingredient | Weight % Active |
|---|---|
| Water | **Balance** |
| Thickener | **1.5** |
| Surfactant | **As Indicated** |
| Chelator | **0.1** |
| Sodium Benzoate | **0.5** |
| Fragrance | **0.9** |
| Petrolatum | **2.0** |
| Stearic Acid | **0.2** |
| Glycerine | **7.0** |
| Sodium Chloride | **0.5** |
| NaOH | **0.1** |
| Polyethylene glycol | **0.0 to 15%** |

The surfactant that may be used includes any of those describe in the specification, and especially, sodium lauryl ether sulfate having 1 EO group derived from carbon obtained from carbon capture. The surfactant may include any combination described in the specification and may especially include a betaine, sodium lauryl ether sulfate and/or acyl isethionate, with or without nonionic surfactant and/or polyethylene, and optionally, any additional surfactants described herein.

It is expected that head space assessment of fragrance that can be made in wash compositions according to the invention will improve when compared to wash compositions made from petrochemicals and with no carbon obtain from carbon capture.

The formulae in these Examples may be made by mixing ingredients with moderate shear, under atmospheric conditions and at temperatures from about 50 to 75°C, with the exception that the temperature should be reduced to about 45°C when fragrance is added. The resulting formulae that may be obtained are expected to have a pH of 6.5 and a water activity at about 0.96. Each formula that may be made can be split and weighed into 5 different aliquots. The 5 different aliquots may then be inoculated with a respective organism or organism mix as identified below, to result in challenged formulations. Inoculum should be equal to 1% of the formula weight and at initiation should not alter the character of the formula being challenged. All formulae should be thoroughly mixed manually after inoculation to distribute the respective microorganisms uniformly. The challenged formulations may then be stored at about 25°C for the duration of the test. Surfactant may be added to base at the amounts indicated.

### Antimicrobial Key

Pool 1: *Pseudomonas aeruginosa* + *Burkholderia cepacia (gram negative)*
Pool 2: *Klebsiella pneumoniae* + *Enterobacter gergoviae (gram negative)*
*3: Staphylococcus aureus* (Staph. aur.)
*4: Candida albicans* (Yeast)
*5: Aspergillus brasiliensis* (Mold)

Complete Eradication ("CE") -destruction/kill of all microbes present.

### Surfactant Key

SLES: Sodium Lauryl Ether Sulfate, 1 EO*
SLS Sodium Lauryl Sulfate
SLI: Sodium Lauroyl Isethionate
SLMI: Sodium Lauroyl Methyl Isethionate
CAPB: Cocoamidopropyl Betaine
Taurate: Sodium Methyl Lauroyl Taurate
*the sodium lauryl ether sulfate, 1 EO suitable for use can have 100% of the ethoxylate portion with carbon obtained from carbon capture.

Challenged formulations may then be sampled for viable microorganisms at 7 days and 14 days. These samples can be plated in a petri dish and incubated under conventional conditions appropriate for such test organisms and as identified in the American Type Culture Collection (ATCC), with the middle point of ranges selected when criteria was defined by a range. After incubation, the number of microbial colonies can be counted, and the resulting figure can be multiplied by the appropriate dilution factor to obtain the number of microorganisms per sample unit.

### Example 14

All formulae (A to G) may be made and tested with surfactant as described in the invention. All formulae may be made with SLES having 1 EO whereby all of the ethoxylate groups may have carbon obtained from carbon capture.

| **Formula A: 4.1 wt% SLES; 2.9% SLS; 1.0% wt% SLI; 2.5wt% CAPB** | | |
|---|---|---|
| | | |

| **Antimicrobial** | **7 Day** | **14 Day** |
|---|---|---|
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **2.70 Log Reduction** | **3.09 Log Reduction** |

| **Formula B: 5.9 wt% SLES; 4.1 wt% SLS; 1.4 wt% SLI; 3.6 CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **3.87 Log Reduction** | **CE** |

| **Formula C: 1.9 wt% SLES; 1.4 wt% SLS; 0.5 wt% SLMI; 1.2 CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **2.49 Log Reduction** | **2.28 Log Reduction** |

| **Formula D: 4.6 wt% SLES; 3.4 wt% SLS; 0 wt% SLI; 2.5 wt% CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **3.34 Log Reduction** | **CE** |

| **Formula E: 3.5 wt% SLES; 2.5 wt% SLS; 2.0 wt% SLI; 2.5 wt% CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **3.34 Log Reduction** | **3.87 Log Reduction** |

| **Formula F: 2.3 wt% SLES; 1.7 wt% SLS; 4.0 wt% SLI; 2.5 wt% CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **3.11 Log Reduction** | **4.04 Log Reduction** |

| **Formula G: 4.1 wt% SLES; 2.9 wt% SLS; 1.0 wt% SLMI, 2.5 wt% CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **3.26 Log Reduction** | **3.87 Log Reduction** |

| **Formula H: 4.1 wt% SLES, 2.9 wt% SLS, 1wt% Glycinate, 2.5 wt% CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **3.04 Log Reduction** | **3.44 Log Reduction** |

| **Formula I: 4.1 wt% SLES, 2.9 wt% SLS, 1 wt% Taurate, 2.5% CAPB** | | |
|---|---|---|
| **Antimicrobial** | **7 Day** | **14 Day** |
| **Pool 1** | **CE** | **CE** |
| **Pool 2** | **CE** | **CE** |
| **Staph. aur.** | **CE** | **CE** |
| **Yeast** | **CE** | **CE** |
| **Mold** | **2.93 Log Reduction** | **3.50 Log Reduction** |

The formulas that may be made according to this invention may be stored for 2 or 3 months at 45°C. After visual assessment by trained panelists, it is expected that none of the formulas displayed will have any negative color changes (browning) or phase separation, i.e., syneresis, especially when compared to identical formulae that do not comprise surfactant having carbon obtained from carbon capture.

Additionally, formulas that may be made according to this invention (i.e., with ingredient having carbon obtained from carbon capture), should unexpectedly, display antimicrobial stability even when they were formulated with sodium benzoate at a pH over the natural pH of skin and with less than 11.0% by weight sulfate-based surfactant. Therefore, the present invention is expected to yield stable formulas that can be made with mild surfactant systems and with preservatives which may be naturally derived. Such stability is expected even when compared to identical formulae that do not comprise surfactant having carbon obtained from carbon capture.

### Example 15

The compositions below may be made with SLES having carbon obtained from carbon capture.

### Shampoo wash compositions 1 and 2

| **Raw Material** | **% Activity** | **Amount in product (wt %, by total composition)** | |
|---|---|---|---|
| | | **1 pH 4.4** | **2 pH 5.8** |
| Sodium Laureth Sulfate | 70 | 17.14 | 17.14 |
| Cocamidopropyl Betaine | 30 | 5.33 | 5.33 |
| Guar Hydroxypropyltrimonium Chloride | 100 | 0.25 | 0.25 |
| Dimethiconol | 22 | 1.6 | 1.6 |
| Glycerin | 100 | 1 | 1 |
| Disodium EDTA | 100 | 0.05 | 0.05 |
| Sodium Hydroxide | 50 | 0.02 | 0.02 |
| Glucono delta lactone | 100 | 0.4 | 0 |
| Sodium sulfate powder | 100 | 0.1 | 0 |
| Trehalose | 100 | 0.1 | 0 |
| Citric acid | 50 | 1 | 1 |
| Sodium chloride | 100 | 1.3 | 1.3 |
| Water & minors (fragrance, pigments, preservative) | - | To 100 | To 100 |
| pH | | 4.4 | 5.8 |

The compositions are expected to be stable, have excellent sensory and have excellent fragrance characteristics, even when prepared with surfactant having carbon obtained from carbon capture.

## Claims

1. A wash composition comprising:
(a) anionic surfactant, nonionic surfactant or both;
(b) amphoteric surfactant, zwitterionic surfactant or both
(c) 0 to 55% by weight polyol; and
(d) water
wherein the anionic surfactant is from 10 to 100% by weight alkyl ether sulfate, acyl isethionate or a mixture thereof based on total weight of anionic surfactant and the nonionic surfactant is from 10 to 100% alcohol ethoxylate based on total weight of nonionic surfactant and further wherein at least 10% by weight of the total weight of anionic and/or nonionic surfactant used comprises carbon derived from carbon capture, the wash composition having a water activity under 1, a pH from 4 to 7.25, and preferably 4.2 to 7.25, and most preferably, from the pH of skin to 7.25 (or from 4.5 to 6.85 or from 5.0 to 6.75 or from 5.5 to 6.5).

2. The wash composition according to claim 1 further comprising polyol and from 0.01 to 15% by weight of a polyether having at least 10% by weight of the total weight of polyether with carbon derived from carbon capture.

3. Use of anionic surfactant, nonionic surfactant or both to make a stable shampoo, shampoo and conditioner, hand wash, face wash or body wash, wherein at least one of the anionic surfactant and nonionic surfactant comprises carbon derived from carbon capture.

4. Use of a polyether, and anionic surfactant, nonionic surfactant or a mixture thereof to make a stable shampoo, hair conditioner, hand wash, face wash or body wash, wherein at least one of the anionic surfactant and nonionic surfactant comprise carbon derived from carbon capture and the polyether comprises carbon derived from carbon capture.

5. Use of a wash selected from a shampoo, shampoo and conditioner, hand wash, face wash or body wash, the wash comprising an anionic surfactant and/or nonionic surfactant comprising carbon derived from carbon capture, and optionally, polyether comprising carbon derived from carbon capture to wash hair or skin on the body.

6. Use of a surfactant and/or other ingredient comprising carbon derived from carbon capture to make a fast-moving cosmetic consumer product.

7. A method for making a wash composition comprising, in no particular order, the steps of combining to produce a mixture:
a) at least one anionic surfactant and/or nonionic surfactant comprising carbon derived from carbon capture, the anionic surfactant comprises alkyl ether sulfate, an acyl isethionate or a mixture thereof and the nonionic surfactant comprises alcohol ethoxylate;
b) amphoteric or zwitterionic surfactant or both;
c) 0 to 55% by weight polyol;
d) 0 to 15% by weight of a polyether; and
e) water
wherein the resulting mixture is mixed or sheared to produce a homogeneous emulsion and further wherein at least one of the anionic surfactants and/or the nonionic surfactant comprises carbon derived from carbon capture, preferably both, and the polyether comprises carbon derived from carbon capture.

8. Use of or method of washing or treating a surface with a wash which is a shampoo, shampoo and conditioner, hand wash, face wash or body wash comprising an anionic surfactant and/or nonionic surfactant comprising carbon derived from carbon capture, and optionally, polyether comprising carbon derived from carbon capture to wash hair or skin or both on the body.

9. A wash composition that is made by a process that uses as an ingredient a surfactant or polyether or both that includes carbon derived from carbon capture.

10. The wash composition according to claims 1 or 2 wherein the wash composition is a shampoo, shampoo and conditioner, hand wash, face wash or body wash.

11. The wash composition according to claim 1 or 2, wherein the wash composition further comprises phenoxyethanol with or without capryloyl glycine and/or undecylenoyl glycine; iodopropynyl butylcarbamate; benzoic acid and/or a derivative of benzoic acid or a mixture thereof and further wherein the wash composition is substantially free of isothiazolinones, hydantoins and parabens and comprises less than 25 ppm heterocyclic impurities comprising 1,4-dioxane.

12. The wash composition according to claim 1 or 2, comprising alkyl ether sulfate, wherein the alkyl ether sulfate comprises an alkyl portion having from 25 to 100%, and preferably, from 35 to 90%, and most preferably, from 50 to 85% by weight C₁₆-C₁₈ alkyl chains based on total weight of alkyl chains and an ether portion (ethylene oxide/EO) having from 25 to 100%, and preferably, from 35 to 90%, and most preferably, from 50 to 85% by weight ether portion with 4 to 7 or 4 to 6 or 4 to 5 ethylene oxide (EO) groups based on the total weight of ether portion.

13. The wash composition according to claim 1 or 2, comprising alkyl ether sulfate wherein the alkyl ether sulfate is sodium lauryl ether sulfate with 1, 2 and 3 poly (ethylene oxide) units.

14. The wash composition according to claim 1 or 2, wherein the isethionate comprises sodium lauroyl isethionate, and the composition further comprises a glycinate comprising sodium lauroyl glycinate, and a taurate comprising sodium methyl lauryl taurate.

15. The wash composition according to claim 1 or 2, wherein the zwitterionic surfactant comprises cocoamidopropyl betaine and further wherein the anionic surfactant which is an isethionate is from 0.4 to 4% by weight of the composition and the composition further comprises 12-hydroxystearic acid, 10-hydroxystearic acid, thiamidol, niacinamide or a mixture thereof.
